# EUROPEAN PATENT APPLICATION

(11) **EP 0 640 338 A1**
(43) Date of publication of application: **01.03.1995**
(21) Application number: 94306293.5
(22) Date of filing: 25.08.1994
(51) Int. Cl.: A61K 31/35

(54) **Sodium cromoglycate for the treatment of upper respiratory tract infections**

(30) Priority: 26.08.1993 GB 9317752
(71) Applicant: FISONS plc, Ipswich Suffolk IP1 1QH (GB)
(72) Inventor: Aberg, Nils Göran, S-436 39 Askim (SE)
(74) Representative: Jones, Stephen Anthony

(57) **Abstract**

The present invention relates to the use of sodium cromoglycate in the therapeutic treatment of the symptoms of upper respiratory tract infections (eg the common cold and influenza).

## Description

This invention relates to the use of sodium cromoglycate in the therapeutic treatment of upper respiratory tract infections.

The term "upper respiratory tract infections" (URTIs) is used by those skilled in the art to describe those acute respiratory illnesses, including the common cold and influenza, which are caused by a variety of different viruses.

Such illnesses are characterised by their sudden onset, and highly infectious and self-limiting nature, with symptoms varying in severity depending upon the virus which is responsible.

Symptoms of the common cold typically start with a mild malaise, fatigue, a dry or sore throat and nasal discomfort, usually reaching maximum severity on the second or third day. At this stage additional, more incapacitating symptoms such as nasal discharge and congestion, sneezing, hoarseness and a cough are observed. In more severe cases patients experience fever with associated headache and muscle pain, vocal impairment and loss of the senses of smell and taste.

Symptoms of influenza are usually more intense and include fever, which may be severe and persist for several days, general fatigue and weakness, and severe discomfort due to intense muscular aching and headache.

Although the symptoms of URTIs are typically not regarded as serious, they often result in lost time at work or school and in this respect impose a substantial cost on society. In addition, even mild URTIs have been known to lead to more serious conditions such as bronchitis; influenza is known to be fatal when acquired by vulnerable individuals.

Existing treatments which find utility in the symptomatic treatment of URTIs comprise active ingredients which target individual or closely related symptoms.

Treatments indicated for overall relief of the symptoms of URTI are thus merely combinations of these active ingredients.

Thus a treatment indicated for relief of all the symptoms of an URTI would typically comprise an analgesic (eg aspirin or paracetamol) to combat fever and relieve pain; a decongestant (eg ephedrine or pseudoephedrine) to reduce nasal congestion; an antitussive (eg codeine) or expectorant (eg ammonium chloride) to alleviate cough; and an antihistamine (eg diphenhydramine or triprolidine) for use as a sedative.

Treatments indicated for sore throats are usually administered separately and may comprise an anaesthetic agent (eg benzocaine) to relieve pain.

There is thus no treatment presently available, comprising a single active ingredient, which is effective against all the recognised symptoms associated with URTIs. All the aforementioned agents are known to exhibit adverse side effects. Moreover, it is the opinion of a substantial proportion of those skilled in the art that some of the treatments mentioned above have little to no positive therapeutic effect.

UK Patent No. 1144905 discloses a number of bis-chromon-2-carboxylic acids, including 1,3-bis(2-carboxy-chromon-5-yloxy)-propan-2-ol, the disodium salt of which, known as sodium cromoglycate, has been known for many years for the prophylactic treatment of chronic respiratory and allergic conditions including, *inter alia*, asthma and allergic rhinitis.

Sodium cromoglycate has a remarkably good safety profile and is known to be relatively free of side effects.

The mode of action of sodium cromoglycate in the prophylaxis of the above conditions is not completely understood. It is, however, known to stabilise mast cell membranes, preventing the release of inflammatory mediators (eg. histamine).

Sodium cromoglyacte is known to have no intrinsic antihistaminic or anti-inflammatory action and is widely considered to possess no bronchodilator activity.

Thus, it is hitherto well known in the art that sodium cromoglycate has no value in the therapeutic treatment of the symptoms of those conditions for which it is known to have preventative efficacy. For example, it is of no value in the treatment of acute asthma (see eg Martindale, The Extra Pharmacopoeia, 29th Edition, at page 1419). nor in the treatment of allergic rhinitis once the symptoms have appeared (see eg Immunology and Allergy Practice, **11**, 151 (1989) and Ars. Medici (Switzerland), **82**, 45 (1990)).

Certain compounds disclosed in UK Patent No. 1144905 were found to enhance the neutralising capacity of the antiserum in certain virus/antibody neutralisation systems. It was therefore postulated that the compounds could find use in the [prophylactic] treatment of viral infections.

In vitro studies by Pentinnen *et al* (BMJ (1977) **1**, 82) found that sodium cromoglycate, in concentrations of 1000 times those active in the stabilisation of mast cell membranes, demonstrated a limited degree of prevention or delay of the appearance of typical cytopathic effects caused by adenovirus 5, echovirus 6 and 7 and herpes simplex virus. However, later studies by Loveday *et al* (BMJ, (1977) **2**, 577) failed to confirm these results and moreover unequivocally demonstrated that sodium cromoglycate demonstrated no antiviral activity against orthomyxovirus and paramyxoviruses, which are associated with influenza and parainfluenza respectively.

Sodium cromoglycate has been shown to give no significant relief in non-allergic eosinophilic rhinitis (Simons *et al*, Clin. Rev. Allergy, **2**, 237 (1984)).

Nebulised solutions of sodium cromoglycate, when administered as a prophylactic treatment, to children with recurrent URTIs were found to exhibit no significant advantage over placebo (water) (Vasamo *et al*, Helv. Paediat Acta, **38**, 335 (1983)). It was concluded that humidification (ie. the use of aqueous solutions *per se*, eg. nebuliser solutions or nose drops) may have some beneficial prophylactic effect on reducing the recurrence rate of recurrent URTIs.

European Patent Application 0 370 362 discloses a solution of sodium cromoglycate in combination with the β₂-adrenoceptor sympathomimetic bronchodilator salbutamol, which finds utility in reversible obstructive airways diseases, including the following: allergic asthma, intrinsic asthma, bronchitis and the nasal and bronchial obstructions associated with the common cold.

Reversible obstructive airways diseases are known by those skilled in the art to be preexisting chronic conditions associated with hyperreactive individuals (ie those with bronchial hyperresponsiveness). It is well known in the art that URTIs *per se* are not reversible obstructive airways diseases. Furthermore, bronchial obstructions are never observed in non-asthmatic individuals who contract an URTI and as such are not associated with URTIs *per se*.

Nevertheless it is also well known that URTIs are potent precipitants of asthma in hyperreactive patients and in particular children (wherein the condition is often termed "wheezy bronchitis"). These exacerbations may in some instances lead to a serious, sometimes fatal, obstruction of the airways (ie nasal and bronchial) both by enhanced inflammation and mucal plugging.

In the wider context of reversible obstructive airways diseases, therefore, nasal and bronchial obstructions associated with URTIs would be understood by one skilled in the art to relate to a worsening of the hyperreactive condition in an asthmatic patient, and not to the mucal secretions and associated congestion confined to the upper respiratory tract which are experienced by otherwise healthy individuals when they contract an URTI.

Moreover, despite the fact that sodium cromoglycate in combination with bronchodilators has been used in the prophylaxis of such exacerbations, when used on its own, the former has been found to exhibit distinctly reduced efficacy (see eg Arch. Dis. Child. **60**, 736 (1985) and Allergy, **41**, 266 (1986)).

Nedocromil sodium, a drug which is also indicated in the prophylactic treatment of asthma and nasal allergy, is known to inhibit mast cell degranulation in a manner similar to sodium cromoglycate. The former, however, also exhibits a much broader range of antiinflammatory action than the latter, affecting the inflammatory response at several sites, including alveolar macrophages and basophils, and has therefore been indicated in nonimmunological challenges such as challenge by sulphur dioxide or sulphites.

Barrow *et al*, Clin. Exp. Allergy, **20**, 45 (1990) and Smith *et al*, J. Psychopharmacology, **5,** 251 (1991) have reported challenge studies in which the prophylactic efficacy of nedocromil sodium was assessed in volunteers who had colds induced by rhinovirus and coronavirus. The trials demonstrated that nedocromil sodium, when administered prior to infection, was partially effective in reducing the development of some of the symptoms of colds caused by rhinovirus (nasal secretion and psychomotor performance) and to a lesser extent coronavirus (psychomotor performance only). The effectiveness of the drug against development of other aforementioned symptoms of URTIs was not mentioned.

The authors concluded that one or more of the mediators affected by nedocromil sodium may play a minor role in the symptoms of URTIs, despite the fact that such mediators have not been detected in individuals infected with URTIs (see eg Acta Otolaryngol, **413** (Suppl), 23 (1984) and Clin Allergy (1988) **18**, 119).

Notwithstanding the latter point, the mucosal mast cell stabilising potency of sodium cromoglycate is a mere 1% that of nedocromil sodium (Eur. J. Respir. Dis. **69**, 112, (1986)). Without being limited by theory, this latter point may account for the fact that, unlike nedocromil sodium, sodium cromoglycate was found to exhibit no prophylactic efficacy in URTIs (see Helv. Paediat Acta, **38**, 335 (1983) *supra*).

In summary, therefore, there is no indication in the prior art that sodium cromoglycate is potentially useful in the therapeutic treatment of the symptoms of URTIs.

Surprisingly we have now found that sodium cromoglycate is highly effective in the therapeutic alleviation of the symptoms of URTIs.

According to the invention there is provided the use of sodium cromoglycate as active ingredient in the manufacture of a medicament for the therapeutic treatment of an URTI.

By "therapeutic treatment of an URTI" we mean treatment of the symptoms of an URTI following recognition of commencement thereof, as distinct from the prophylaxis of such a condition.

Particular URTIs which may be mentioned include the common cold and influenza.

In particular we have found that sodium cromoglycate when administered therapeutically may relieve fatigue, fever and nasal and bronchial congestion; reduce the volume of nasal secretion and the incidence and severity of sneezing and cough; and relieve sore throats and vocal impairment experienced by patients suffering from URTIs.

Thus, according to another aspect of the invention there is provided a method of treating a human having an URTI which comprises administering a therapeutically effective quantity of a medicament comprising sodium cromoglycate to a patient suffering from such a condition.

The method of treatment according to the invention may find utility in the alleviation of symptoms of URTIs in hyperreactive patients and non-hyperreactive patients. However, we prefer a method of treatment of a non-hyperreactive patient.

By "a non-hyperreactive patient" we mean a patient who does not exhibit abnormal sensitivity to spasmodic stimuli resulting in recurrent, variable or intermittent narrowing of intrapulmonary airways (eg a non-asthmatic patient).

Formulations comprising sodium cromoglycate for the treatment of URTIs may be administered topically by a variety of routes, for example intra-nasally or by oral inhalation.

According to a further aspect of the invention there is provided a pharmaceutical formulation for the therapeutic treatment of an URTI, which contains a therapeutically effective quantity of sodium cromoglycate.

Dealing first with oral inhalation, sodium cromoglycate may be inhaled as a dry powder in formulations which may be pressurized or non-pressurized.

In non-pressurized powder formulations, the active ingredient may be pelletised, eg as described in UK Patent No 1520247, or in finely divided form. When the active ingredient is in finely divided form it may be used in admixture with a larger sized pharmaceutically acceptable inert carrier comprising particles, of up to 100µm diameter. Suitable inert carriers include sugars, for example crystalline lactose.

The powder may be administered from a capsule containing the active ingredient using an inhalation device. The capsule may contain from 5 to 40mg, for example 10 to 30mg and preferably 15 to 25mg of the active ingredient.

Inhalation devices which may be employed include the Spinhaler™. Powder formulations may also be inhaled from a pre-pierced capsule, eg. as disclosed in International Patent Application No. PCT/GB93/01909, using a device such as that disclosed in International Patent Application No. PCT/GB94/00388.

Alternatively powder formulations may be inhaled directly from a disposable device such as that disclosed in European Patent No. 0 404 454.

The formulation may alternatively be pressurized and contain a compressed gas, eg nitrogen, or a liquefied gas propellant.

Suitable propellants are the chlorofluorocarbons sold under the Trade Mark "Freon", which are non-toxic and have a boiling point below 20°C at atmospheric pressure. Examples of these propellants are dichlorodifluoromethane, 1,2-dichlorotetrafluoroethane and trichloromonofluoromethane. It may be preferred however, in view of environmental concerns, to use one of the hydrofluoroalkane propellants, for example, 1,1,1,2-tetrafluoroethane, 1,1-difluoroethane or 2H-heptafluoropropane. Mixtures of the above mentioned propellants may suitably be employed.

The pressurized formulation may also contain a surface active agent. The surface active agent may be a liquid or solid non-ionic surface active agent (eg sorbitan trioleate) or may be a solid anionic surface active agent. It is preferred to use the solid anionic surface active agent in the form of the sodium salt, for example sodium dioctyl-sulphosuccinate, provided that the propellant is not a hydrofluoroalkane.

Commercially available pressurised formulations of sodium cromoglycate include INTAL® Inhaler (1mg and 5mg).

Formulations suitable for administration by nasal inhalation include solutions, especially aqueous solutions.

When the sodium cromoglycate is to be used in aqueous solution, it may be necessary to incorporate a tonicity modifying agent (for example sodium chloride or glycerol) to reduce irritation on administration of the active ingredient. In addition the solution may also contain an effective proportion of a pharmaceutically acceptable chelating or sequestering agent (for example sodium edetate or sodium carboxymethyl cellulose). The solution may also, if desired, contain an effective proportion of a pharmaceutically acceptable preservative or sterilising agent (eg. quaternary ammonium salts, especially that known generically as 'Benzalkonium chloride'). In addition the solution may also contain a buffering agent (eg. sodium hydrogen phosphate or sodium phosphate).

The solution may contain from 0.1 to 10%, preferably from 0.5 to 8% of active ingredient. We particularly prefer solutions which contain from 1 to 6% of active ingredient.

Typical single doses may contain from 1 to 20mg, for example 2 to 10mg and preferably 3 to 8mg of the active ingredient.

Sodium cromoglycate may be formulated along with other active ingredients indicated for the treatment of URTIs, for example sympathomimetic agents commonly used as decongestants (eg. ephedrine, oxymetazoline, phenylephrine and xylometazoline). However, only α-adrenoceptor sympathomimetic agents are presently used as decongestants. We therefore prefer formulations which do not contain β-adrenoceptor sympathomimetic agents, and in particular β₂-adrenoceptor sympathomimetic agents (eg clenbuterol, fenoterol, pirbuterol, reproterol, rimiterol, salbutamol, salmeterol, terbutaline and trimetoquinol). Thus, we prefer formulations which are substantially free of β₂-adrenoceptor sympathomimetic agents.

By "formulations which are substantially free of β₂.adrenoceptor sympathomimetic agents" we mean formulations wherein the level of the such agents is insufficient to have an effect on the relief of the symptoms of URTIs, for example less than 0.001% w/v.

However, in view of the widely recognised side effects which are exhibited with conventional active ingredients indicated for the treatment of URTIs, we prefer formulations wherein sodium cromoglycate is present as the sole active ingredient, ie. formulations which consist essentially of sodium cromoglycate.

By "formulations which consist essentially of sodium cromoglycate" we mean formulations which are essentially free of other active ingredients such that the level of the latter is insufficient to have an effect on the relief of the symptoms of URTIs, for example less than 0.001% w/v, and that other components may also be present in the formulation, provided that the essential characteristics of the formulation are not materially affected by their presence.

The mode of administration depends upon the symptoms to be treated.

Where the symptom to be treated comprises one or more of nasal congestion, nasal secretion, sneezing, fever and general weakness, we prefer administration to the nose.

Where the symptom to be treated comprises one or more of cough, sore throat, vocal impairment, fever and general weakness, we prefer administration via oral inhalation.

For complete treatment of all the symptoms of URTIs however, we prefer a method of treatment which comprises either separate, simultaneous or sequential adminstration of a formulation adapted for oral inhalation, in conjunction with a formulation adapted for nasal inhalation.

Thus, according to a further aspect of the invention, there is provided a pharmaceutical product containing a first formulation of sodium cromoglycate adapted for administration to the nose and a second formulation of sodium cromoglycate adapted for oral inhalation, as a combined preparation for separate, simultaneous or sequential use in the therapeutic treatment of an upper respiratory tract infection.

The dosage administered will naturally depend *inter alia* on the mode of adminstration, and also the severity of the URTI.

In general, we have found that in order to treat the symptoms of URTIs, sodium cromoglycate should be administered at a frequency of, for example between a half hourly and twelve hourly basis, and particularly between a one hourly and four hourly basis.

Alternatively, sodium cromoglycate may be administered more frequently (eg. on a half hourly to three hourly basis) over the first few days (eg. day one to day three) of the URTI and less frequently (eg. three to twelve hourly basis) thereafter until complete relief of symptoms.

The method of treatment according to the invention has the advantage that sodium cromoglycate may be used to alleviate all the recognised symptoms of URTIs. Further sodium cromoglycate is less toxic, is more efficacious, is longer acting, has a broader range of activity, is more potent, produces fewer side effects, or has other useful pharmacological properties, compared with active ingredients previously used in the treatment of URTIs.

The invention is illustrated by the following examples:

### Example 1

### Formulation for nasal adminstration

Commercially available sodium cromoglycate nasal spray (5.2mg per dose; 4% solution, RYNACROM®)

### Example 2

### Formulations for oral inhalation

(a) Commercially available capsules of sodium cromoglycate dry powder blended with lactose (20mg dose; SPINCAPS®)
(b) Commercially available capsules of sodium cromoglycate dry powder in pelletised form (20mg dose; SPINCAPS®)
(c) Commercially available multi-dose inhaler containing CFC's 12/114 (1mg or Smg dose; INTAL®)
(d) Sodium cromoglycate dry powder blended with lactose (as Example 2(a); 10 to 30mg doses) are contained in a pre-pierced capsule as described in International Patent Application No. PCT/GB93/01909.
(e) Sodium cromoglycate dry powder in pelletised form (as Example 2(b); 10 to 30mg doses) are contained in a pre-pierced capsule as described in International Patent Application No. PCT/GB93/01909.
(f) Sodium cromoglycate powder blended with lactose (as Example 2(a); 10 to 30mg doses) are contained in an embodiment of the disposable device disclosed in European Patent No. 0 404 454.
(g) Sodium cromoglycate powder in pelletised form (as Example 2(b); 10 to 30mg doses) are contained in an embodiment of the disposable device disclosed in European Patent No. 0 404 454.

### Example 3

### Pharmaceutical Combination Pack

The above formulations may be used separately, simultaneously or sequentially according to enclosed instructions in the following manner:
(a) The nasal spray of Example 1 was used in combination with the capsules of Example 2(a) via the commercially available Spinhaler® device.
(b) The nasal spray of Example 1 is used in combination with the capsules of Example 2(b) via the commercially available Spinhaler® device.
(c) The nasal spray of Example 1 is used in combination with the multi-dose inhaler of Example 2(c).
(d) The nasal spray of Example 1 is used in combination with sodium cromoglycate contained in the pre-pierced capsules of Example 2(d). Sodium cromoglycate is inhaled orally from the pre-pierced capsule using an embodiment of the device disclosed in International Patent Application No. PCT/GB94/00388.
(e) The nasal spray of Example 1 is used in combination with sodium cromoglycate contained in the pre-pierced capsules of Example 2(e). Sodium cromoglycate is inhaled orally from the pre-pierced capsule using an embodiment of the device disclosed in International Patent Application No. PCT/GB94/00388.
(f) The nasal spray of Example 1 is used in combination with sodium cromoglycate contained in the disposable device of Example 2(f) from which drug is directly inhaled.
(g) The nasal spray of Example 1 is used in combination with sodium cromoglycate contained in the disposable device of Example 2(g) from which drug is directly inhaled.

### Example 4

### Clinical Trial

A randomised, double blind, group comparative, placebo-controlled study was conducted at two centres in order to establish the absolute treatment efficacy of sodium cromoglycate in URTIs.

The study was conducted according to Good Clinical Trial Practice, to the Applicant's standard operating procedures, and to the principles of the Declaration of Helsinki (as modified in Hong Kong).

Patients between 21 and 63 (mean 41) years of age were recruited on a voluntary basis. In view of the fact that URTIs are short lived, patients were asked to enter the study as soon as possible. Patients with URTI symptoms which they had had for more than 24 hours were therefore excluded from the trial.

In order to provide a sample of patients with no significant disease other than URTIs, and who took no other medication which could potentially mask the true effect of sodium cromoglycate, the following patients were also excluded from the trial:
(a) those with a history of
   (i) bronchial asthma;
   (ii) allergic or vasomotor rhinitis;
   (iii) clinically relevant immunological deficiency;
   (iv) chronic bronchitis;
   (v) nasal polyps;
   (vi) chronic cough;
   (vii) gastro-intestinal disease;
   (viii) cardiac conditions
   (ix) renal disease; and
   (x) endocrine disease;
(b) pregnant or lactating patients or those at risk of pregnancy (ie. those using contraception not considered adequate by the investigator);
(c) those having received recognised asthma and/or allergy drugs, ie:
   (i) corticosteroids;
   (ii) sodium cromoglycate;
   (iii) nedocromil sodium;
   (iv) inhaled bronchodilators;
   (v) anticholinergics;
   (vi) theophyllines; and
   (vii) sympathomimetics
   up to four weeks prior to the trial; and
(d) those having received other specified medication, ie:
   (i) antihistamines;
   (ii) non-steroidal antiinflammatory drugs; and
   (iii) antibiotics
   up to four weeks prior to the trial.

Patients were divided into two groups. The first group of patients inhaled one capsule of sodium cromoglycate dry powder (20mg dose) via the Spinhaler®, along with one dose of sodium cromoglycate nasal spray (5.2mg per dose; 4% solution) to each nostril, on a two-hourly basis (except at night time) during the first 48 hours of treatment (Day 1 to Day 3 of the trial). Thereafter treatment was continued at the same dose levels four times a day until the complete relief of URTI symptoms was achieved, up to a maximum of seven days (ie up to Day 8 of the trial).

The second group was treated with placebo (lactose) dry powder via the Spinhaler® and placebo (as sodium cromoglycate nasal spray, but excluding active ingredient), in exactly the same fashion as the first group.

Upon recruitment patients were instructed how to use the above treatments and to record symptoms on a diary card, which was completed on a twice daily basis (morning and evening). Patients were asked to score the following URTI symptoms based on the indicated criteria:
1. General Weakness
   0 = None
   1 = Feeling a little tired
   2 = Wanting to go to bed
   3 = Wanting to sleep
2. Sore Throat
   0 = None
   1 = Slight irritation
   2 = Moderately sore
   3 = Difficult to swallow and/or sleep
3. Sneezing
   0 = None
   1 = 1-5 times a day
   2 = 6-15 times a day
   3 = More than 15 times a day
4. Nasal Secretion
   0 = None
   1 = Had to blow nose 1-5 times a day
   2 = Had to blow nose 6-15 times a day
   3 = Had to blow nose more than 15 times a day
5. Freezing (chill)
   0 = None
   1 = Feeling a little frozen/chill
   2 = Single agues
   3 = Multiple agues
6. Nasal Congestion
   0 = None
   1 = Intermittent
   2 = Almost permanent
   3 = Patient only able to breath through mouth
7. Ache/Pain
   0 = None
   1 = Headache; pain in the chest or back; or pain in arm(s) or legs(s)
   2 = Aches/pains in any two of the above
   3 = Aches/pains in all of the above
8. Cough
   0 = None
   1 = 1-5 attacks a day
   2 = More than 5 attacks a day
   3 = Prolonged attacks
9. Vocal Distortions
   0 = Normal
   1 = Coarse
   2 = Hoarse
   3 = Cannot Speak

Symptom scores were analysed with parametric statistical methods in two steps. A two-way (drug*time) ANOVAs for repeated measures was applied first. If there were significant interaction effects, the scores were analysed post-hoc using Student's t-tests as the second step. Two-tailed tests were used. Statistical significance of results originating from two-way tables was assessed using a χ-squared test or Fisher's exact test.

At the end of the trial, patients were also asked to give an overall efficacy rating for the treatment they had received according to the following criteria:
1 = Highly effective
2 = Moderately effective
3 = Slightly effective
4 = No effect
5 = Made the condition worse

Patients were also asked to guess whether or not they had received active substance or placebo.

### Results

### In all, 135 subjects were initially randomised to treatment. Of the original number, 7 were later found not to have met the eligibility criteria, 9 were uncooperative and 1 experienced an adverse effect which was not related to the drug. Data from 118 was thus available for analysis (54 in the active group and 64 in the placebo). About 10 subjects appeared with a clinical picture of influenza.

The mean scores for each individual symptom on a day-by-day basis are presented graphically in the accompanying figures in which:

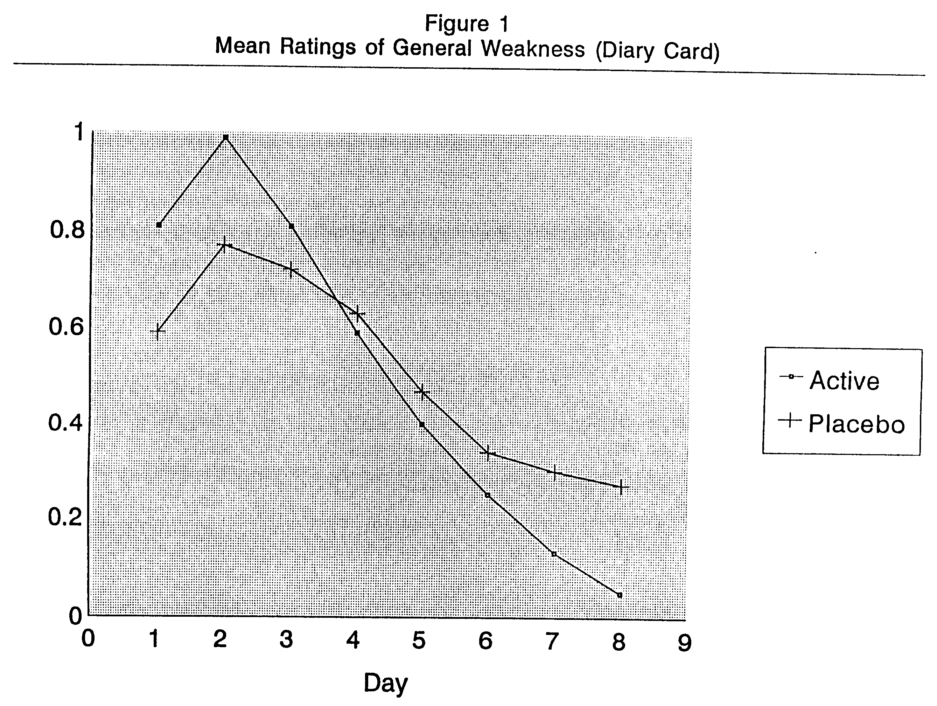

Figure 1 is plot of mean ratings of general weakness (diary card) against day of the trial;

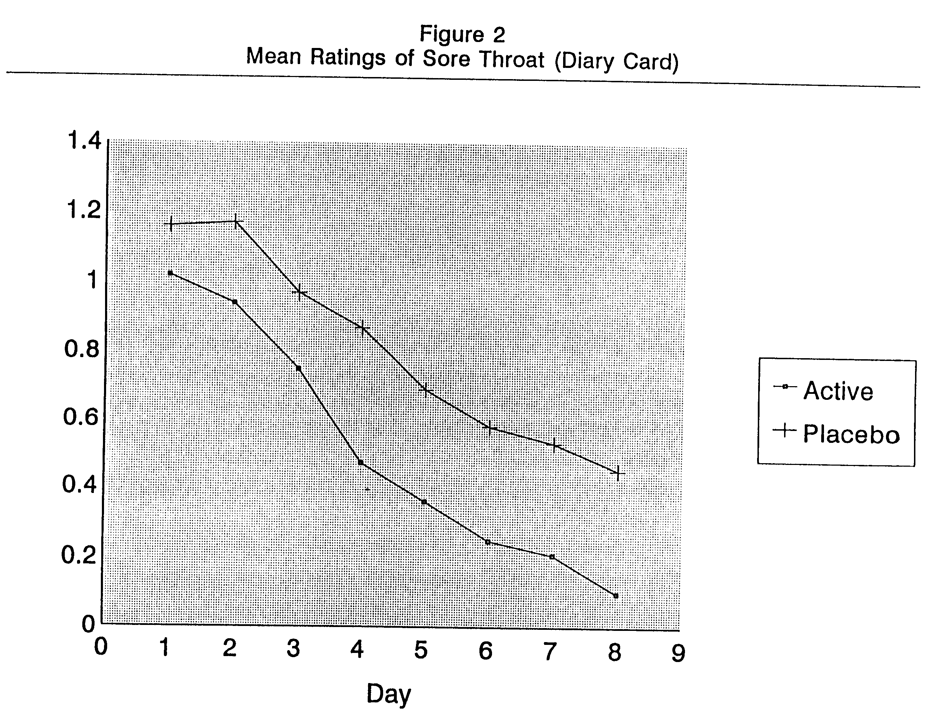

Figure 2 is plot of mean ratings of sore throat (diary card) against day of the trial;

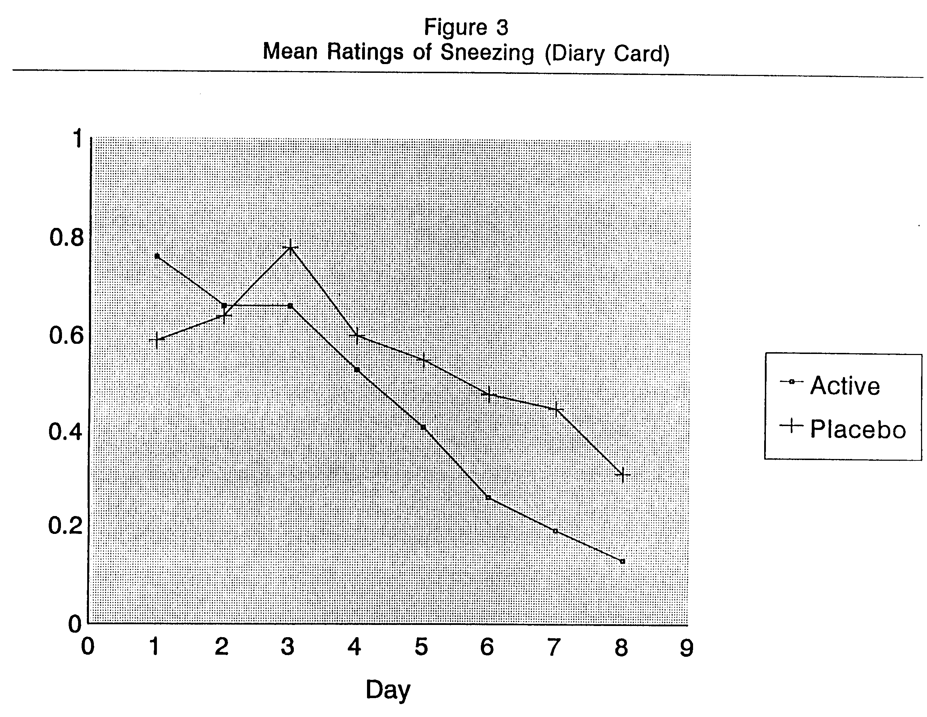

Figure 3 is plot of mean ratings of sneezing (diary card) against day of the trial;

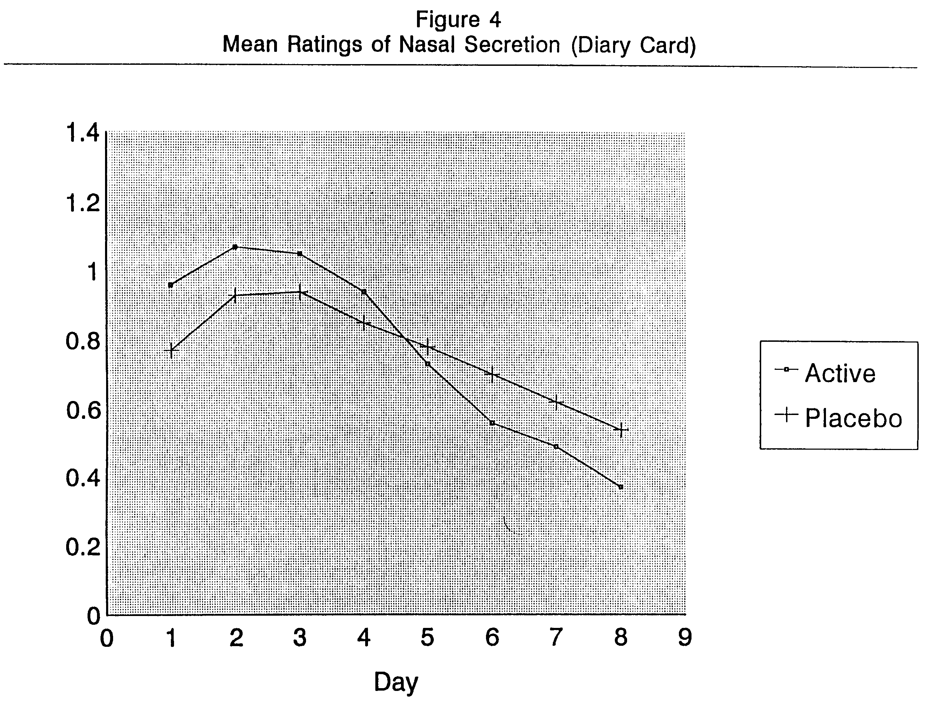

Figure 4 is plot of mean ratings of nasal secretion (diary card) against day of the trial;

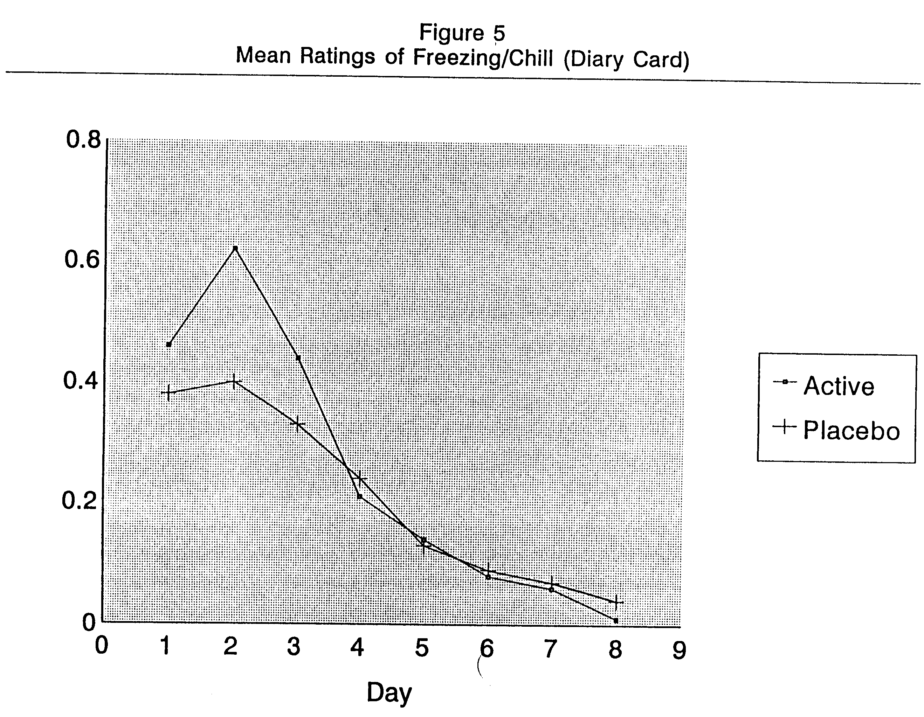

Figure 5 is plot of mean ratings of freezing/chill (diary card) against day of the trial;

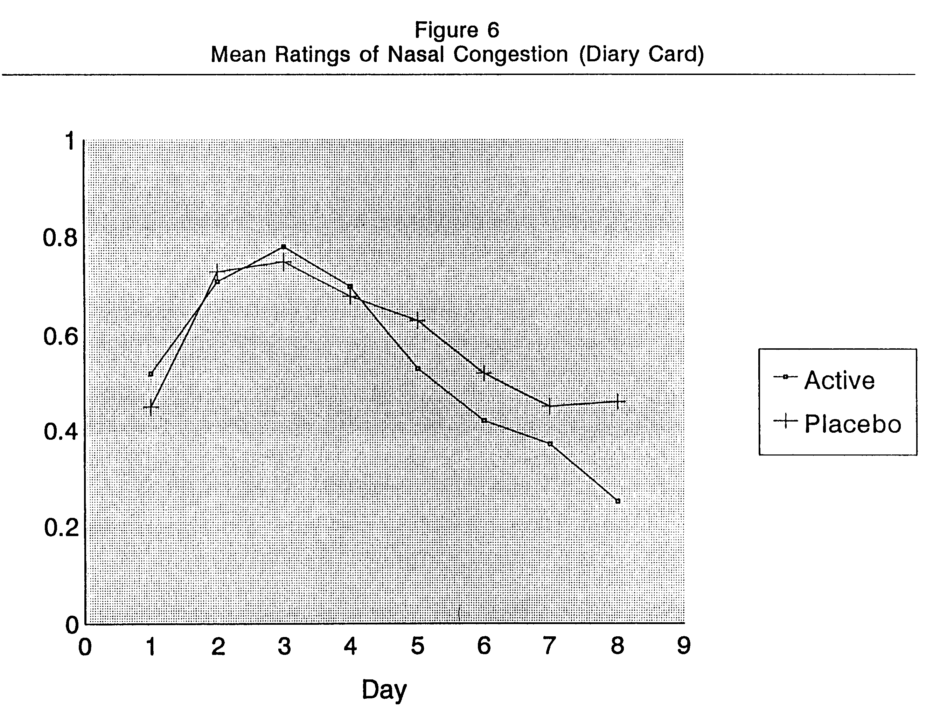

Figure 6 is plot of mean ratings of nasal congestion (diary card) against day of the trial;

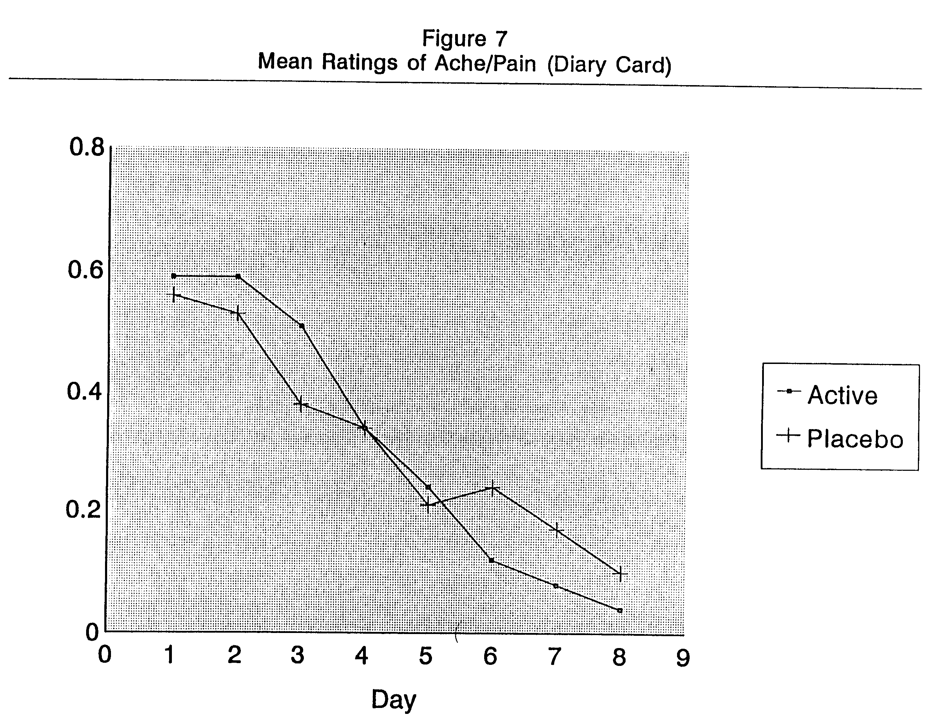

Figure 7 is plot of mean ratings of ache/pain (diary card) against day of the trial;

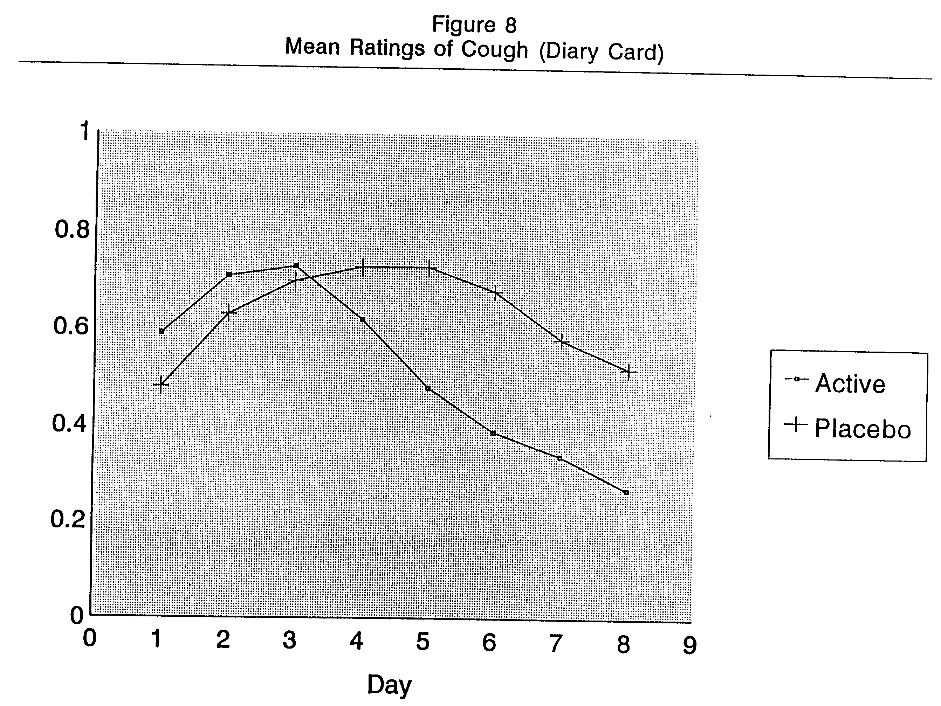

Figure 8 is plot of mean ratings of cough (diary card) against day of the trial;

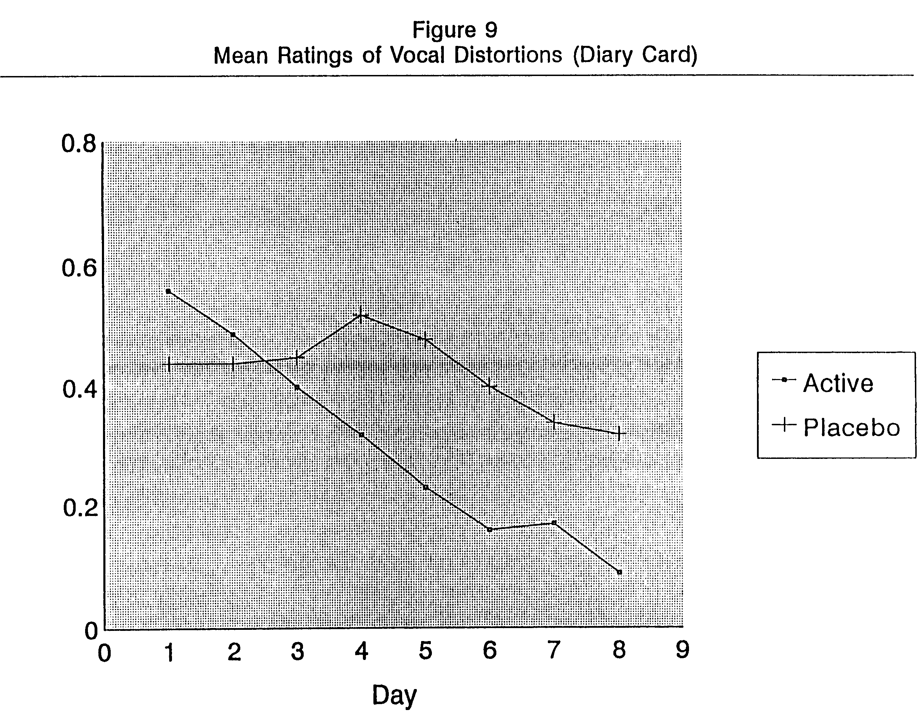

Figure 9 is plot of mean ratings of vocal distortions (diary card) against day of the trial;

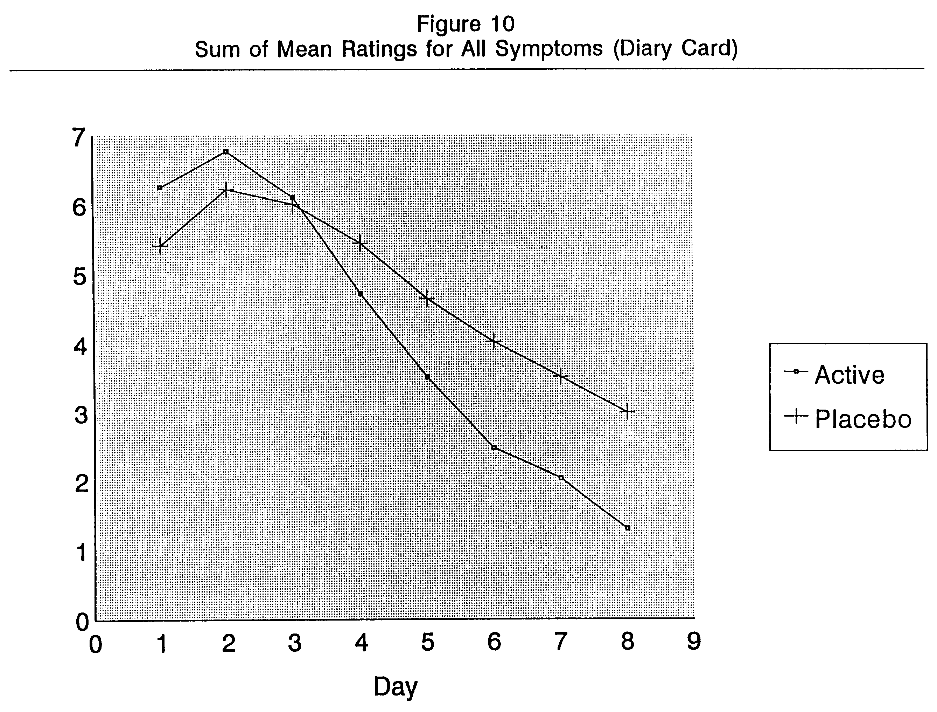

Figure 10 is plot of the sum of the mean ratings for all symptoms (diary card) against day of the trial; and
Figure 11 is plot of the sum of the mean ratings for the "non-fever" URTI symptoms (ie sore throat, sneezing, nasal secretion, nasal congestion, cough and vocal distortions; diary card) against day of the trial.

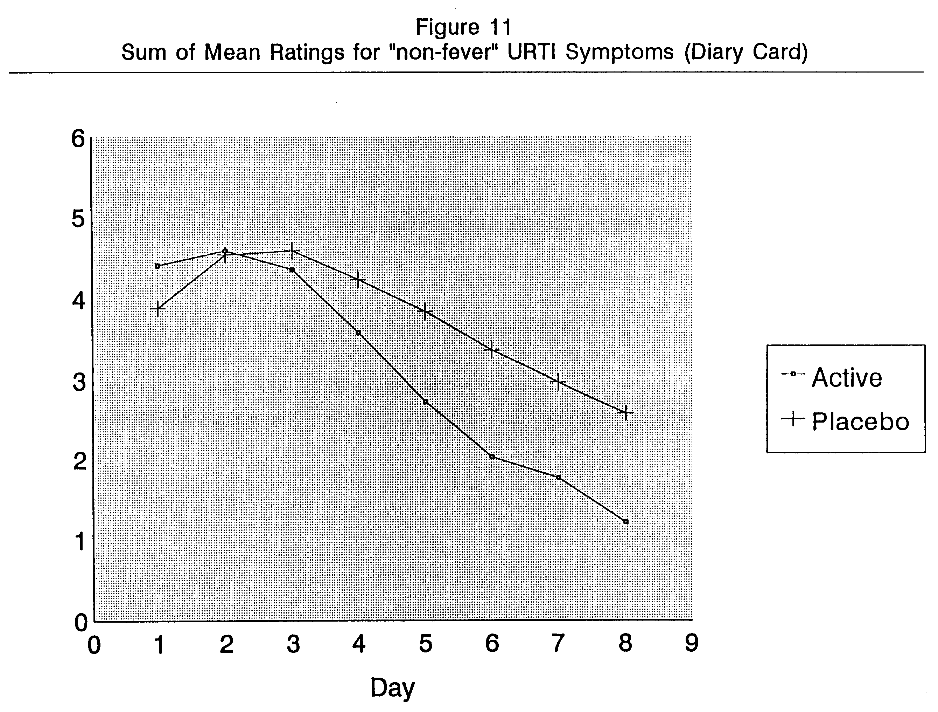

The results clearly demonstrate that sodium cromoglycate, when administered therapeutically significantly speeds up the recovery rate from the symptoms of URTIs. Figure 11 demonstrates that on Day 5 the value of the sum of the mean ratings for the "non-fever" URTI symptoms for the sodium cromoglycate group is the same as that for the placebo group on the Day 8 (post-hoc t-test for Day 7: p≦0.01).

Patients' general opinion of efficacy is tabulated below:

| Opinion | Sodium cromoglycate Group | Placebo Group |
|---|---|---|
| Very Effective | 16 (31%) | 4 (7%) |
| Moderately Effective | 22 (42%) | 9 (15%) |
| Slightly Effective | 9 (17%) | 16 (26%) |
| Had No Effect | 5 (10%) | 32 (52%) |
| Made Condition Worse | 0 (0%) | 0 (0%) |

(Group diff. p<0.001; Five patients refused to give their opinion)

The results clearly favour the sodium cromoglycate group.

Of the subjects asked to guess which treatment they had been receiving, 81 agreed to do so: 91% of the subjects on active treatment guessed that they were on active treatment in comparison with 30% of the placebo group (p<0.001). The value for the placebo group is typical.

## Claims

1. Use of sodium cromoglycate as active ingredient in the manufacture of a medicament for the therapeutic treatment of an upper respiratory tract infection.

2. Use as claimed in claim 1, characterised in that the medicament is substantially free of β₂-adrenoceptor sympathomimetic agents.

3. Use as claimed in claim 1, characterised in that sodium cromoglycate is the sole active ingredient.

4. Use as claimed in any of claims 1 to 3, characterised in that the upper respiratory tract infection is the common cold.

5. Use as claimed in any of claims 1 to 3, characterised in that the upper respiratory tract infection is influenza.

6. Use as claimed in any of claims 1 to 5, characterised in that the treatment is of a non-hyperreactive patient.

7. Use as claimed in any of claims 1 to 6, characterised in that the medicament is adapted for administration to the nose.

8. Use as claimed in any of claims 1 to 6, characterised in that the medicament is adapted for oral inhalation.

9. Use as claimed in any of claims 1 to 6, characterised in that the medicament comprises a first formulation of sodium cromoglycate adapted for administration to the nose and a second formulation of sodium cromoglycate adapted for oral inhalation.

10. A pharmaceutical product containing a first formulation of sodium cromoglycate adapted for administration to the nose and a second formulation of sodium cromoglycate adapted for oral inhalation, as a combined preparation for separate, simultaneous or sequential use in the therapeutic treatment of an upper respiratory tract infection.

11. A pharmaceutical product as claimed in claim 10, characterised in that the formulations are substantially free of β₂-adrenoceptor sympathomimetic agents.

12. A pharmaceutical product as claimed in claim 10, characterised in that the formulations contain sodium cromoglycate as the sole active ingredient.

13. A pharmaceutical product as claimed in any of claims claim 10 to 12, characterised in that the treatment is of a non-hyperreactive patient.
